# EUROPEAN PATENT APPLICATION

(11) **EP 4 074 262 A1**
(43) Date of publication of application: **19.10.2022**
(21) Application number: 20900393.8
(22) Date of filing: 07.12.2020
(51) Int. Cl.: A61B 10/00, B01L 3/00

(54) **SPECIMEN CONTAINER**

(30) Priority: 12.12.2019 KR 20190166106
(71) Applicant: Park, Je Hyun, Gwangju 61501 (KR)
(72) Inventor: IM, Young Duk, Gwangju 61735 (KR); PARK, Je Hyun, Gwangju 61501 (KR)
(74) Representative: Modiano, Gabriella Diana
(86) International application number: PCT/KR2020/017740
(87) International publication number: WO 2021/118183

(57) **Abstract**

The present invention relates to a sample container having a new structure to effectively transfer a sample. A sample container according to the present invention includes: a container main body 10 that is formed in a cup shape having an open top surface, and a coupling hole 11 opened in a vertical direction is formed on one side of the container main body 10; a closed and sealed cover 20 that is coupled to an upper end of the container main body 10 to close and seal the container main body 10; and a detachably coupled collection tube 30 that is formed in the shape of a tube that extends in a vertical direction while having an open top surface, and is detachably coupled to the coupling hole 11, wherein a slope 12 that is inclined downward to the inside of the container main body 10 is formed on one side of an external circumferential surface of the container main body 10, wherein a recess portion 13 of which a lower end is open, and the recess portion 13 is formed on one side of the external circumferential surface of the container main body 10, and wherein the coupling hole 11 is formed to penetrate top and bottom surfaces of the recess portion 13, and the coupling hole 11 is formed on the top surface of the recess portion 13, accordingly, there is a merit that, after containing urine in the container main body 10 and combining the closed and sealed cover 20, the container main body 10 can be tilted to easily transfer urine to the collection tube 30 and the urine can be prevented from outflowing and contaminating the surrounding area, being contaminated, or being mistakenly switched.

## Description

### [Technical Field]

The present invention relates to a sample container having a new structure to effectively transfer samples to a collection tube.

### [Background Art]

In general, when performing a urine test, a testee provides urine as a sample in a sample container of an appropriate size, and then it is transferred to a collection tube in the form of a test tube with a small diameter for examination. However, when the urine contained in the sample container is transferred to the collection tube as described above, urine may be spilled by a worker's mistake, contaminating the surrounding area, and causing loss of urine. In addition, when there are multiple urine samples, a mistake such as transferring the urine contained in the sample container to different collection tubes may occur. In particular, a bar code indicating testee information should be attached to the sample container provided to the testee and the collection tube containing urine, respectively. In this case, a mistake that the sample is changed due to incorrect attachment of the bar code may occur. Such a problem is not limited to the sample container used to contain the urine described above, but the same may occur in a container storing blood or other various types of liquid samples. Therefore, there is a need for a new method that can solve such a problem.

### [Disclosure]

### [Technical Problem]

The present invention is to solve the above problem, and its purpose is to provide a sample container of a new structure that can effectively transfer and contain a sample in a collection tube.

### [Technical Solution]

To achieve the above-stated purpose, the present invention provides a sample container that includes: a container main body 10 that is formed in a cup shape having an open top surface, and a coupling hole 11 opened in a vertical direction is formed on one side of the container main body 10; a closed and sealed cover 20 that is coupled to an upper end of the container main body 10 to close and seal the container main body 10; and a detachably coupled collection tube 30 that is formed in the shape of a tube that extends in a vertical direction while having an open top surface, and is detachably coupled to the coupling hole 11, wherein a slope 12 that is inclined downward to the inside of the container main body 10 is formed on one side of an external circumferential surface of the container main body 10, wherein a recess portion 13 of which a lower end is open, and the recess portion 13 is formed on one side of the external circumferential surface of the container main body 10, and wherein the coupling hole 11 is formed to penetrate top and bottom surfaces of the recess portion 13, and the coupling hole 11 is formed on the top surface of the recess portion 13.

According to another feature of the present invention, the container main body 10 is provided with an extension flap 16 covering the upper part of the coupling hole 11.

According to another feature of the present invention, the extension flap 16 is inclined downward to the inside.

According to another feature of the present invention, the recess portion 13 is formed between a support plate 14 that extends to the inside on an interior circumference of the container main body 10 and where the coupling hole 11 is formed, and a diaphragm 15 that extends downward at a circumferential portion, the extension flap 16 extends upward from an upper end of the container main body 10, and a base end is formed to form an arc shape with both ends bent inward when viewed from the top, and when the extension flap 16 is folded inward, the extension flap 16 is extended inward and fixed to cover an upper part of the coupling hole 11.

### [Advantageous Effects]

The sample container according to the present invention includes: a container main body 10 that is formed in a cup shape having an open top surface, and a coupling hole 11 opened in a vertical direction is formed on one side of the container main body 10; a closed and sealed cover 20 that is coupled to an upper end of the container main body 10 to close and seal the container main body 10; and a detachably coupled collection tube 30 that is formed in the shape of a tube that extends in a vertical direction while having an open top surface, and is detachably coupled to the coupling hole 11, wherein a slope 12 that is inclined downward to the inside of the container main body 10 is formed on one side of an external circumferential surface of the container main body 10, a recess portion 13 of which a lower end is open is formed on one side of the external circumferential surface of the container main body 10, and the coupling hole 11 is formed to penetrate top and bottom surfaces of the recess portion 13, and the coupling hole 11 is formed on the top surface of the recess portion 13, accordingly, there is a merit that, after containing urine in the container main body 10 and combining the closed and sealed cover 20, the container main body 10 can be tilted to easily transfer urine to the collection tube 30 and the urine can be prevented from outflowing and contaminating the surrounding area, being contaminated, or being mistakenly switched.

### [Description of the Drawings]

FIG. 1 is a side cross-sectional view of a sample container according to the present invention.
FIG. 2 is a side cross-sectional view that illustrates a disassembled state of the sample container according to the present invention.
FIG. 3 is a top plan view of a container main body of the sample container according to the present invention.
FIG. 4 is a normal cross-sectional view that illustrates the cross-section of a line A-A of FIG. 1.
FIG. 5 is a referential view illustrating operation of the sample container according to the present invention.
FIG. 6 is a side cross-sectional view of a variation of the sample container according to the present invention.
FIG. 7 is a side cross-sectional view of a second embodiment of the sample container according to the present invention.
FIG. 8 is a top plan view of a sample container according to the second embodiment of the sample container according to the present invention.
FIG. 9 is a side cross-sectional view of the second embodiment of the sample container according to the present invention.
FIG. 10 is a side cross-sectional view of a variation of the second embodiment of the sample container according to the present invention.

### [Mode for Invention]

Hereinafter, the present invention will be described in detail with the accompanying drawings.

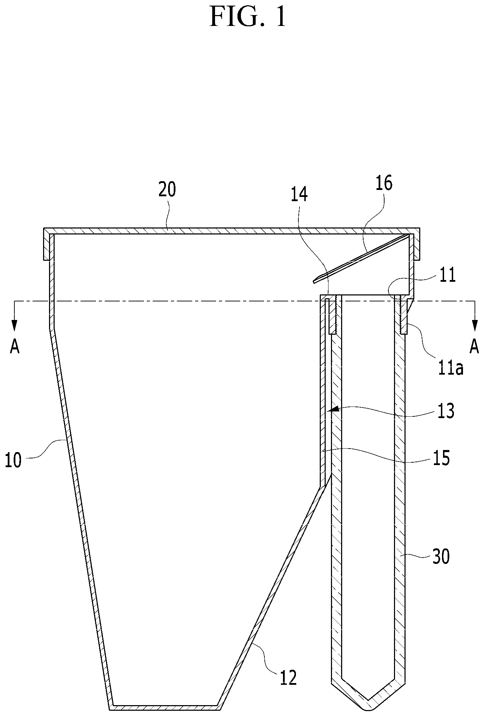

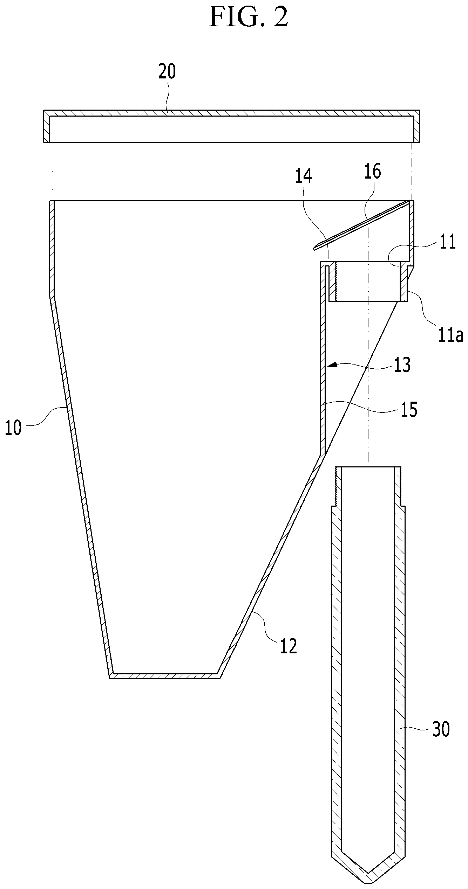

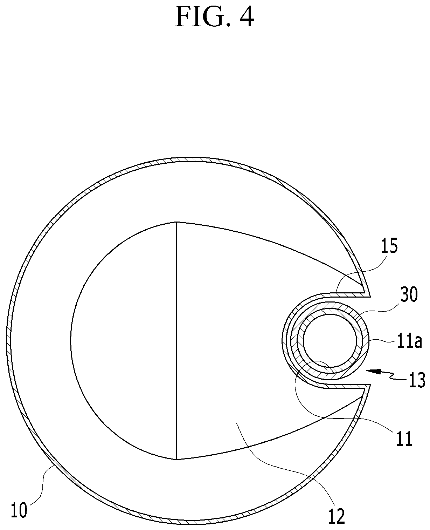

FIG. 1 to FIG. 5 illustrate a sample container, which is used to store urine.

According to the drawings, the sample container is formed of a container main body 10 with a cup shape having an open top and including a coupling hole 11 opened in a vertical direction on one side, a closed and sealed cover 20 coupled to the upper end of the container main body 10 to close and seal the container main body 10, and a detachably coupled collection tube 30 formed in the shape of a tube extending in a vertical direction and having an open top surface, and of which an upper end is detachably coupled to the coupling hole 11.

The container main body 10 is formed of a synthetic resin material that is transparent and has adequate elasticity, and a slope 12 inclined downward to the inside of the container main body 10 and a recess portion 13 are formed on one side of the external circumferential surface of the containing main body 10.

The slope 12 inclined downward to the inside of the container main body 10 is formed on one side of the external circumferential surface of the container main body 10.

For example, the slope 12 formed on one side of the external circumferential surface of the container main body 10 and inclined downward to the inside of the container main body 10 may be formed to be in contact with a lower part, specifically, a lower end, of a diaphragm 15 forming the recess portion 13.

For example, the slope 12 formed on one side of the external circumferential surface of the container main body 10 and inclined downward to the inside of the container main body 10 may be connected with one end of a plane that is parallel to the horizontal plane, and the one end and the other end may be formed to be in contact with the lower part, specifically, the lower end of the diaphragm 15 forming the recess portion 13.

The slope 12 is formed by forming the circumference surface of one side of the container main body 10 in a downwardly inclined plane shape.
It is described that the slope 12 is formed by forming the circumference surface on one side of the container main body 10 to a plane inclined downward as an example, but the slope 12 may be formed in various shapes such as a curved surface by forming one circumference surface of the container main body 10 to be inclined downward to the inside of the container main body 10.

The recess portion 13 is formed between a support plate 14, where the coupling hole 11 is formed, that extends to the inside on an interior circumference of the container main body 10 and a diaphragm 15 that extends downward at a circumferential portion, and the collection tube 30 can be inserted thereinto.

A lower end of the recess portion 13 may be open. Specifically, the lower end of the recess portion 13 may be open to the slope 12.

The coupling hole 11 is formed in the support plate 14 to penetrate the upper and lower surfaces of the support plate 14, and a fixed end pipe 11 a extending downward is provided on the lower part of a support hole.

In addition, the container main body 10 is provided with an extension flap 16 covering the upper part of the coupling hole 11.

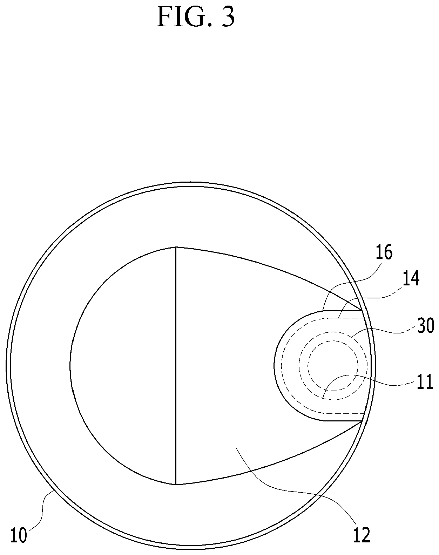

The extension flap 16 extends downward from the upper end of the container main body 10 to the inside of the container main body 10. As shown in FIG. 3, a width of the lateral direction and a width of inner and outer directions are slightly wider than a width of the lateral direction and a width of inner and outer directions of the support plate 14 and thus that the entire upper part of the support plate 14 can be covered, and thus when a testee urinates, the urine can be prevented from flowing directly into the collection tube 30 through the coupling hole 11, and at the same time, when urine hits the upper surface of the extension flap 16, urine splashes inside the container main body 10 so that it is collected inside the container main body 10.

The closed and sealed cover 20 is tightly coupled to the outside of the upper end of the container main body 10 to thereby close and seal the upper end of the container main body 10.

The collection tube 30 is formed in the shape of a test tube that extends in a vertical direction and has an open upper end, and the upper end thereof is air-tightly coupled to the inside of the fixed end pipe 11a.

In this case, the upper end of the collection tube 30 is screwed into the interior circumference of the fixed end pipe 11a.

In addition, a length of the collection tube 30 in the vertical direction is longer than a length of the recess portion 13 in the vertical direction, and thus when the collection tube 30 is coupled to the fixed end pipe 11a of the coupling hole 11, the lower end of the collection tube 30 extends long to the lower part of the slope 12.

A method of using the sample container formed in such a manner will now be described.

First, the collection tube 30 is attached with a bar code that displays testee information.

Then, the testee puts a certain amount of urine in the container main body 10 to which the collection tube 30 is combined, and then closes and seals the upper end of the container main body 10 by combining the closed and sealed cover 20.

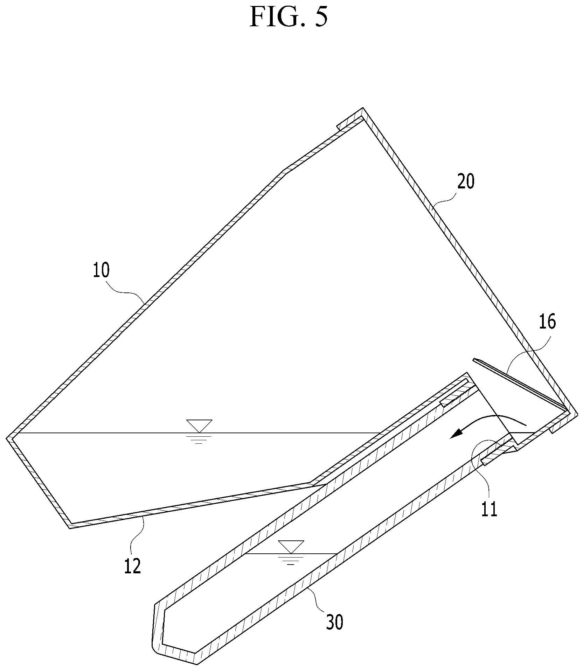

Next, as shown in FIG. 5, when the container main body 10 is tilted toward the recess portion 13, urine stored in the container main body 10 flows into the collection tube 30 through the coupling hole 11.

In addition, the collection tube 30 is separated from the coupling hole 11, and the upper end of the collection tube 30 is sealed and closed with a separate stopper such that the urine can be contained in the collection tube 30.

A sample container formed as described above includes a container main body 10 with a cup shape having an open top and including a coupling hole 11 open in a vertical direction on one side, a closed and sealed cover 20 coupled to the upper end of the container main body 10 to close and seal the container main body 10, and a detachably coupled collection tube 30 formed in the shape of a tube extending in a vertical direction and having an open top surface, and of which an upper end is detachably coupled to the coupling hole 11, and a slope 12 is formed inclined downward to the inside of the container main body 10 on one side of the external circumferential surface of the containing main body 10.

A diaphragm 15 concave inward may be formed on the external circumferential surface of the container main body 10 to intersect the slope 12.

A recess portion 13 with an opened lower end is formed on one side of the external circumferential surface of the container main body 10 and the coupling hole 11 is formed on a top surface of the recess portion 13 to penetrate the top and bottom surfaces of the recess portion 13, and accordingly, there is a merit that, after containing urine in the container main body 10 and combining the closed and sealed cover 20, the container main body 10 can be tilted to easily transfer urine to the collection tube 30 and the urine can be prevented from flowing and contaminating the surrounding area, being contaminated, or being mistakenly switched.

In addition, an extension flap 16 covering the upper part of the coupling hole 11 is provided in the container main body 10, and thus, when a testee urinates, the urine discharged initially can be prevented from flowing into the collection tube 30 through the coupling hole 11 and can be stored.

In particular, the extension flap 16 is inclined downward to the inside of the container main body 10, and thus when the urine hits the extension flap 16, it then bounces into the container main body 10 and is stored, thereby preventing the urine from bouncing out after hitting the extension flap 16.

In case of the present embodiment, the sample container according to the present invention is described to be used to contain urine, but the sample container according to the present invention can be used to contain samples of various types of liquids other than urine.

In addition, it has been described that the width of the lateral direction and the width of inner and outer directions are slightly wider than the width of the lateral direction and the width of inner and outer directions of the support plate 14 as an example, but the width of the lateral direction and the width of inner and outer directions of the extension flap 16 may be narrow enough to cover only the top of the coupling hole 11.

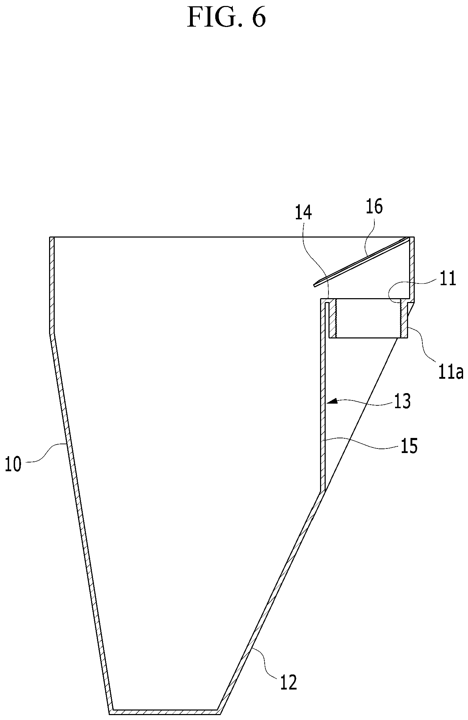

In addition, it has been described that the extension flap 16 extends downward from the upper end of the container main body 10 to the inside of the container main body 10 as an example, but as shown in FIG. 6, the extension flap 16 may extend into the inside of the container main body 10 at an interior circumference positioned slightly below the upper end of the container main body 10.

In addition, it has been described the upper end of the collection tube 30 is screwed into the interior circumference of the fixed end pipe 11a as an example, but the upper end of the collection tube 30 may be fitted into the interior circumference of the fixed end pipe 11a, or screwed or fitted into the external circumferential surface of the fixed end pipe 11a.

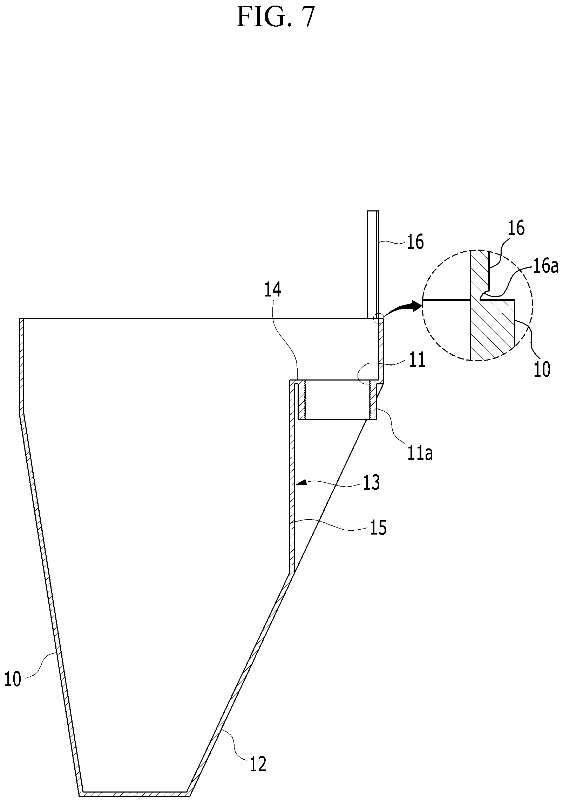

FIG. 7 to FIG. 9 illustrate another embodiment of the present invention, wherein an extension flap 16 extends upward from an upper end of the container main body 10, and thus when the extension flap 16 is folded inward, the extension flap 16 is extended inward and fixed to cover an upper part of a coupling hole 11.

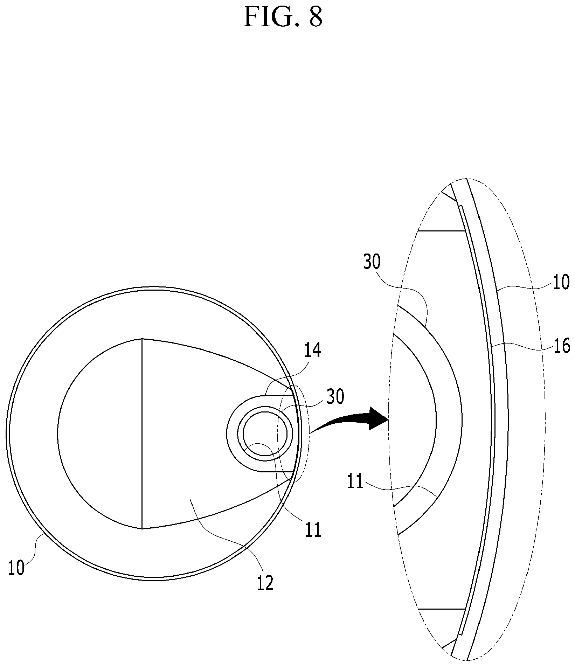

To this end, the extension flap 16 has a thinner sheet shape than that of the container main body 10, and, when a base end is viewed from the top, as shown in FIG. 8, both ends are bent inward while forming an arc shape.

In addition, the extension flap 16 has a length in the vertical direction that is longer than a distance from a lower end of the extension flap 16 to a front end of a support plate 14, and, as shown in FIG. 7, a fold 16a, which is thinner than the extension flap 16, is formed at a connection between the lower end of the extension flap 16 and the upper end of the container main body 10.

In this case, the extension flap 16 is formed so that the interior circumference thereof extends from the interior circumference of the container main body 10.

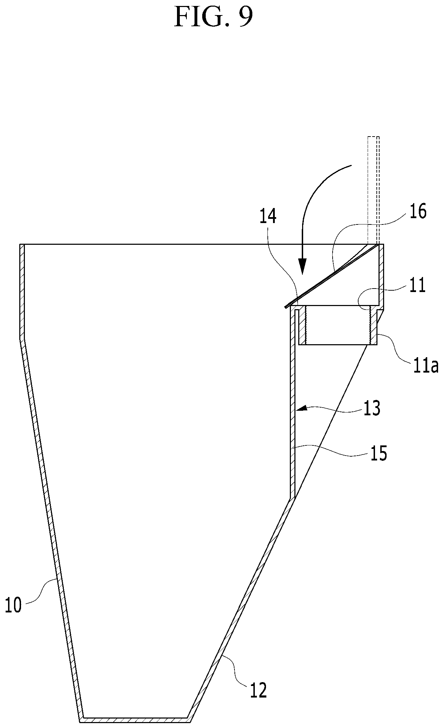

Thus, as shown in FIG. 9, when the extension flap 16 is folded inward, the extension flap 16 is in a state where the base end is fixed to the upper end of the container main body 10, and a front end portion is bent so that both ends are upward such that the lower part of the front end portion of the extension flap 16 adheres to an upper surface of an inner end of the support plate 14 of the recess portion 13.

That is, the base end of the extension flap 16 forms an arc shape with both ends bent inward, and thus when the extension flap 16 is folded inward, a a force that pulls a middle part of the extension flap 16 (a middle part between both ends) back is generated, and accordingly, a force causing the front end of the extension flap 16 to rotate downward generates such that the extension flap 16 is fixed not to lift upward while the front end of the extension flap 16 is in close contact with the upper surface of the inner end of the support plate 14.

In the sample container formed as described above, the extension flap 16 extends upward from the upper end of the container main body 10 and the base end is formed in the shape of an arc with both ends bent inward when being viewed from the top, and thus when the extension flap 16 is folded inward, the end of the extension flap 16 is fixed to be contact with the circumference of the upper surface of the support plate 14.

Therefore, it is easy to manufacture the sample container, and there is a merit that the cost required for manufacturing the sample container can be reduced.

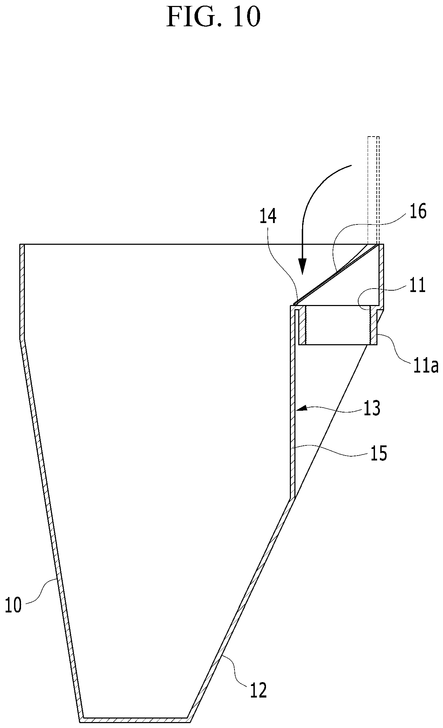

In case of the present embodiment, it is described that the extension flap 16 has a longer length in the vertical direction than a distance from a lower end of the extension flap 16 to a front end of a support plate 14, and thus the extension flap 16 adheres to the upper surface of the inner end of the support plate 14 of the recess portion 13 as an example, but, as shown in FIG. 10, it is also possible to shorten the length of the extension flap 16 so that the front end of the extension flap 16 adheres to the upper surface of the support plate 14, while covering the coupling hole 11.

In addition, the extension flap 16 may be formed such that a lower surface is fixed upwardly separating from an upper surface of the support plate 14.

While this invention has been described in connection with what is presently considered to be practical embodiments, it is to be understood that the invention is not limited to the disclosed embodiments. On the contrary, it is intended to cover various modifications and equivalent arrangements included within the spirit and scope of the appended claims.

## Claims

1. A sample container comprising:
a container main body 10 that is formed in a cup shape having an open top surface, and a coupling hole 11 opened in a vertical direction is formed on one side of the container main body 10;
a closed and sealed cover 20 that is coupled to an upper end of the container main body 10 to close and seal the container main body 10; and
a detachably coupled collection tube 30 that is formed in the shape of a tube that extends in a vertical direction while having an open top surface, and is detachably coupled to the coupling hole 11,
wherein a slope 12 that is inclined downward to an inside of the container main body 10 is formed on one side of an external circumferential surface of the container main body 10,
wherein a recess portion 13 of which a lower end is open, and the recess portion 13 is formed on one side of the external circumferential surface of the container main body 10, and
wherein the coupling hole 11 is formed to penetrate top and bottom surfaces of the recess portion 13 and the coupling hole 11 is formed on a top surface of the recess portion 13.

2. The sample container of claim 1, wherein
the container main body 10 is provided with an extension flap 16 covering an upper part of the coupling hole 11.

3. The sample container of claim 2, wherein the extension flap 16 is inclined downward to an inside.

4. The sample container of claim 2, wherein
the recess portion 13 is formed between a support plate 14 that extends to an inside on an interior circumference of the container main body 10 and where the coupling hole 11 is formed, and a diaphragm 15 that extends downward at a circumferential portion,
the extension flap 16 extends upward from an upper end of the container main body 10, and a base end is formed to form an arc shape with both ends bent inward when viewed from the top, and
when the extension flap 16 is folded inward, the extension flap 16 is extended inward and fixed to cover an upper part of the coupling hole 11.
